# EUROPEAN PATENT APPLICATION

(11) **EP 4 108 784 A1**
(43) Date of publication of application: **28.12.2022**
(21) Application number: 21757111.6
(22) Date of filing: 19.02.2021
(51) Int. Cl.: C12Q 1/6886, C12Q 1/6869, C12Q 1/6876

(54) **MARKER SELECTION METHOD USING METHYLATION DIFFERENCE BETWEEN NUCLEIC ACIDS, METHYLATED OR DEMETHYLATED MARKER, AND DIAGNOSTIC METHOD USING MARKER**

(30) Priority: 20.02.2020 KR 20200020974
(71) Applicant: Eone Diagnomics Genome Center Co., Ltd., Incheon 22014 (KR)
(72) Inventor: LEE, Sunghoon, Incheon 22014 (KR); MIN, Na Young, Incheon 22014 (KR); KWON, Hyukjung, Incheon 22014 (KR); BAE, Jin-Sik, Incheon 22014 (KR); LEE, Min Seob, Incheon 22014 (KR); SHIN, Shang Cheol, Incheon 22014 (KR)
(74) Representative: Schiweck Weinzierl Koch Patentanwälte Partnerschaft mbB
(86) International application number: PCT/KR2021/002149
(87) International publication number: WO 2021/167413

(57) **Abstract**

The present invention relates to a marker screening method using differences in methylation of nucleic acids, a demethylation marker and a diagnostic method using the marker, more specifically, a novel method for screening disease-specific demethylation markers using methylation differences in free nucleic acids, and relates to a new cancer diagnosis method by methylation detection for determining cancer by calculating the demethylation marker and the frequency of the marker screened by this method, and to a cancer-specific demethylation marker in the selected cfDNA.

## Description

### THECHNICAL FIELD

This application claims priority to Korean Patent Application No. 10-2020-0020974 filed on February 20, 2020, and the entire specifications of which are incorporated herein by reference in their entireties.

The present invention relates to a marker screening method using differences in methylation of nucleic acids, a demethylation marker and a diagnostic method using the marker, more specifically, a novel method for screening disease-specific demethylation markers using methylation differences in free nucleic acids, and relates to a new cancer diagnosis method by methylation detection for determining cancer by calculating the demethylation marker and the frequency of the marker screened by this method, and to a cancer-specific demethylation marker in the selected cfDNA.

### BACKGROUND OF THE INVENTION

Cancer refers to a group of abnormal cells generated by continuous division and proliferation by disrupting the balance between cell division and death due to various causes, and is also called a tumor or neoplasia. It usually affects more than 100 different parts of the body, including organs, white blood cells, bones, lymph nodes, etc. and develops into serious symptoms through infiltration into surrounding tissues and metastasis to other organs.

Even today with advances in medical science, the 5-year survival rate for human cancers, particularly solid tumors (cancers other than blood cancer), which accounts for the majority, is less than 50%. About two-thirds of all cancer patients are found at an advanced stage, and most of them die within two years of diagnosis. Such poor therapeutic effect of cancer is not only a matter of treatment method, this is because it's not easy to find methods for diagnosing actual cancer early, diagnosing advanced cancer accurately, and following up after treatment.

In the current clinical setting, cancer diagnosis is conducted through history taking, physical examination, and clinical pathology examination, and once suspected, radiographic examination and endoscopy are performed, finally, confirmed by biopsy. However, with the existing clinical test method, the number of cancer cells must be 1 billion cells and the diameter of the cancer must be 1 cm or more to be diagnosed. In this case, cancer cells already have the ability to metastasize, and in fact, more than half of the cancer has already metastasized. On the other hand, tumor markers that find substances directly or indirectly produced by cancer in the blood are used for cancer screening, but this has a limit in accuracy, so even when there is cancer, it appears normal up to about half, and even when there is no cancer, often appears benign, causing confusion. In addition, in the case of an anticancer agent mainly used for the treatment of cancer, there is a problem in that the effect is shown only when the volume of the cancer is small.

As described above, both diagnosis and treatment of cancer are difficult because there are many differences from normal cells, and it is very complex and diverse. Cancer continues to grow in an excessive amount at will, free from death and continue to survive, invade surrounding tissues and spread (metastasize) to distant organs, causing human death. It survives attacks of immune mechanisms or chemotherapy, continues to evolve, and the most favorable cell group (clones) for survival selectively proliferates. Cancer cells are living organisms with high viability that are caused by mutations in a number of genes. In order for a single cell to turn into a cancer cell and develop into a malignant cancer mass seen in clinical practice, mutations in multiple genes must occur. Therefore, it is necessary to approach at the genetic level in order to fundamentally diagnose and treat cancer.

Accordingly, recently, methods for diagnosing cancer through DNA methylation measurement have been proposed. DNA methylation mainly occurs in the cytosine of the CpG island of the promoter region of a specific gene. As a result, the binding of transcription factors is disturbed and the expression of specific genes is blocked (gene silencing). This is the main mechanism by which the function of the gene is lost without mutation in the protein-specific coding sequence of the gene in vivo, and it has been interpreted as the cause of the loss of functions of a number of tumor suppressor genes in human cancer. There is controversy over whether methylation of the promoter CpG island directly induces carcinogenesis or whether it is a secondary change in carcinogenesis, but such aberrant methylation/demethylation in CpG islands has been reported in various cancer cells including prostate cancer, colon cancer, uterine cancer, and breast cancer, etc. Therefore, it can be used in various fields such as early diagnosis of cancer, prediction of cancer risk, prediction of cancer prognosis, follow-up after treatment, and prediction of response to chemotherapy. Attempts to use it for cancer diagnosis and screening by testing it by methods such as methylation-specific PCR (hereinafter referred to as MSP), automatic sequencing, or bisulfite pyrosequencing have been actively made recently, but many are limited to methods for detecting and analyzing the methylation of a small number of specific genes or promoter regions (for example, Korean Patent No. 1557183, Korean Patent No. 1119947), and there is a limit to the efficiency and accuracy of diagnosis.

In particular, overall methylation changes occur in the genome of cancer cells, and the most extensive changes occur in repeat sequences. There are various types of genome repeat sequences such as transposon, retrotransposon, LINE, and SINE, and they occupy a large proportion to occupy more than half of the entire genome, but research has not been relatively done. The reason is that repeating sequences are difficult to perform functional analysis, do not assemble well, and are easily excluded from analysis because there are many regions not included in the reference sequence (reference standard sequence). For this reason, methylation studies on repeat sequences have not been conducted relatively well, and research on the meaning and development of markers for cancer-related methylation, which occurs frequently in repeat sequences, has not been relatively active. However, in studies using various genomic analysis technologies, various research results have been accumulated that hypomethylation of DNA occurs extensively with cancer progression (Epigenomics. 2009 December ; 1(2): 239-259, Clin Chem Lab Med. 2012 Oct 1;50(10):1733-42), so hypomethylation in repeat sequences is expected to be used as a diagnostic marker for cancer.

Numerous papers and patent documents are referenced throughout this specification and their citations are indicated. The disclosures of the cited papers and patent documents are incorporated herein by reference in their entirety to more clearly describe the level of the technical field to which the present invention pertains and the content of the present invention.

### DETAILED DESCRIPTION OF THE INVENTION

### TECHNICAL PROBLEM

Accordingly, the present inventors were researching to develop a novel method capable of accurately diagnosing cancer in a non-invasive manner, after treating the cfDNA with a methylation-sensitive restriction enzyme to cut and sequencing the non-methylated sequence among the restriction enzyme target sequences. In the case of classifying each sequence using sequence information of a certain length in the decoded sequence, it is possible to classify the types of cfDNA in the blood for diseases, especially diseases such as cancer. Through this, it was confirmed that it can act as a cfDNA marker for diseases, the present invention was completed by developing a method for screening cancer-specific markers, particularly cancer-specific demethylation markers, associated with methylation in cfDNA.

Accordingly, it is an object of the present invention to provide a novel method for screening cancer-specific demethylation markers in cfDNA.

Another object of the present invention is to provide a novel cancer diagnosis method by demethylation detection for determining cancer by calculating the frequency of cancer-specific demethylation markers in screened cfDNA.

Another object of the present invention is to provide a method of sequencing and analyzing sequence information of a predetermined length at the N-terminus of a methylation-sensitive restriction enzyme fragment of cfDNA isolated from blood of a subject to provide information necessary for cancer diagnosis.

Another object of the present invention is to provide a cancer-specific demethylation marker in cfDNA selected by the method of the present invention, wherein the N-terminus of the cancer-specific demethylation marker is the sequence of the cohesive end of the recognition site of a methylation-sensitive restriction enzyme, and consists of a sequence of 25 to 150 bases.

### TECHNICAL SOLUTION

In order to achieve the above object, the present invention provides a method of screening a cancer-specific demethylation marker in cfDNA comprising: treating cfDNA (cell free DNA) isolated from blood of a subject with methylation sensitive restriction enzymes; analyzing the sequence of each fragment; obtaining sequence information of a predetermined length from the N-terminus of the fragment; counting the frequency of each of the sequence information; screening cancer-specific sequence information as a cancer-specific demethylation marker in cfDNA.

In addition, in order to achieve another object of the present invention, the present invention provides a novel cancer diagnosis method by demethylation detection for determining cancer by calculating the frequency of cancer-specific demethylation markers in screened cfDNA.

In order to achieve another object of the present invention, the present invention provides a method of analyzing sequence information of a predetermined length at the N-terminus of a methylation-sensitive restriction enzyme fragment of cfDNA isolated from blood of a subject to provide information necessary for cancer diagnosis.

In order to achieve another object of the present invention, the present invention provides a cancer-specific demethylation marker in cfDNA selected by the method of the present invention, wherein the N-terminus of the cancer-specific demethylation marker is the sequence of the cohesive end of the recognition site of a methylation-sensitive restriction enzyme, and consists of a sequence of 25 to 150 bases.

Unless otherwise defined, all technical and scientific terms used herein have the same meaning as commonly understood by one of ordinary skill in the art. The following references provide one of the skills with general definitions of various terms used in the present specification.: Singleton et al., DICTIONARY OF MICROBIOLOGY AND MOLECULAR BIOLOTY(2th ed. 1994); THE CAMBRIDGE DICTIONARY OF SCIENCE AND TECHNOLOGY(Walkered., 1988); and Hale & Marham, THE HARPER COLLINS DICTIONARY OF BIOLOGY.

Hereinafter, the present invention will be described in detail.

The present invention relates to a method of screening a cancer-specific demethylation marker in cfDNA comprising: (a) treating cfDNA (cell free DNA) isolated from blood of a subject with methylation sensitive restriction enzymes; (b) analyzing the sequence of each fragment; (c) obtaining sequence information of a predetermined length from the N-terminus of the fragment; (d) counting the frequency of each of the sequence information; and (e) screening cancer-specific sequence information as a cancer-specific demethylation marker in cfDNA.

### Methylation

Any nucleic acid in purified or unpurified form in the present invention may be used, and any nucleic acid that contains or is suspected of containing a nucleic acid sequence containing a target site (e.g., a CpG-containing nucleic acid) can be used. The nucleic acid site that can be differentially methylated is the C position of the CpG sequence, and methylation is particularly high in CpG islands where GpG is dense. At certain sites, the density of CpG islands is 10-fold higher compared to other regions of the genome. CpG islands have an average G*C ratio of about 60%, whereas normal DNA exhibits an average G*C ratio of 40%. CpG islands are typically about 1-2 kb in length, and there are about 45,000 CpG islands in the human genome.

Typically, the sample nucleic acid is DNA. However, nucleic acid mixtures may also be used. The specific nucleic acid sequence to be detected may be a fraction of a large molecule, and the specific sequence may exist in the form of isolated molecules constituting the entire nucleic acid sequence from the outset. The nucleic acid sequence need not be a nucleic acid present in pure form, and nucleic acids may be small fractions within a complex mixture, such as containing whole human DNA. The nucleic acid contained in the sample used for measuring the degree of methylation of the nucleic acid contained in the sample or used for detecting the methylated CpG island may be extracted by a conventional method known in the art.

### Sequencing

Sequencing methods include, for example, Sanger sequencing, high-throughput sequencing, pyrosequencing, sequencing by synthesis, single molecule sequencing, nanopore sequencing, semiconductor sequencing, sequencing by ligation, sequencing by hybridization, RNA-Seq (Illumina), digital gene expression (Helicos), Next Generation Sequencing (NGS), Single Molecule Sequencing by Synthesis (SMSS) (Helicose), Massively Parallel Sequencing, Clonal Single Molecule Arrays [Solexa], Shotgun Sequencing, Ion Torrent, Oxford Nanopores, Roche Genia, Maxim-Gilbert sequencing, primer walking; PacBio, SOLiD, Ion Torrent, or sequencing using a nanopore platform. The sequencing reaction may be performed in various sample processing units, which may be multiple lanes, multiple channels, multiple wells, or other means of processing multiple sets of samples substantially simultaneously. The sample processing unit may also include multiple sample chambers that allow simultaneous processing of multiple executions.

The sequencing reaction may be performed on one or more types of nucleic acid, at least one of which is known to contain a marker of a disease. The sequencing reaction may also be performed on any nucleic acid fragment present in the sample.

Simultaneous sequencing reactions can be performed using multiplex sequencing. In some cases, cell-free nucleic acids can be sequenced in at least 1000, 2000, 3000, 4000, 5000, 6000, 7000, 8000, 9000, 10000, 50000, 100,000 sequencing reactions. In other cases, cell-free nucleic acids may be sequenced in less than 1000, 2000, 3000, 4000, 5000, 6000, 7000, 8000, 9000, 10000, 50000, 100,000 sequencing reactions. Sequencing reactions may be performed sequentially or simultaneously. Subsequent data analysis may be performed on all or part of the sequencing reaction. In some cases, data analysis may be performed on at least 1000, 2000, 3000, 4000, 5000, 6000, 7000, 8000, 9000, 10000, 50000, 100,000 sequencing reactions. In other cases, data analysis may be performed on less than 1000, 2000, 3000, 4000, 5000, 6000, 7000, 8000, 9000, 10000, 50000, 100,000 sequencing reactions. An exemplary read depth is 1000-50000 reads per gene locus (base).

### Sample

The sample may be any biological sample isolated from a subject. The sample may be a body sample. The sample may include body tissue, such as a known or suspected solid tumor, whole blood, serum, plasma, feces, leukocytes or lymphocytes, endothelial cells, tissue biopsy, cerebrospinal fluid, synovial fluid, lymphatic fluid, ascites, interstitial fluid or extracellular fluid, fluids in the intercellular spaces (including fluids from the gums), bone marrow, pleura exudate, cerebrospinal fluid, saliva, mucus, sputum, semen, sweat, urine. The sample may be may be further processed that in the form originally isolated from the subject or to remove or add components such as cell or to enrich one component compared to another component. A sample may be isolated or obtained from a subject and transported to a sample analysis site. Samples can be stored and shipped at a desired temperature, for example, room temperature, 4°C, - 20°C, and/or -80°C. A sample may be isolated or obtained from a subject at a sample analysis site.

The subject may be a human, mammal, animal, pet animal, service animal, or pet. The subject may have a disease. The subject cannot be free of the disease or detectable disease symptoms. The individual may have been treated with one or more therapies, for example, any one or more of surgery, treatment, dosing, chemotherapy, antibody, vaccine, or biologics. The subject may or may not be in remission.

### Cell-free nucleic acids in blood samples

A blood sample may contain varying amounts of nucleic acid containing genomic equivalents. For example, a sample of about 33 ng DNA may contain about 10,000 (10⁴) haploid human genome equivalents and, in the case of cfDNA, about 200 billion (2×10¹¹) individual polynucleotide molecules. Similarly, a sample of about 100 ng of DNA may contain about 30,000 haploid human genome equivalents and, in the case of cfDNA, about 600 billion individual molecules.

Exemplary amounts of cell-free nucleic acid in a sample prior to amplification range from about 1 fg to about 1 µg, eg, from 1 pg to 200 ng, from 1 ng to 100 ng, from 10 ng to 1000 ng. For example, the amount may be about 600 ng or less, about 500 ng or less, about 400 ng or less, about 300 ng or less, about 200 ng or less, about 100 ng or less, about 50 ng or less, or about 20 ng or less, or about 10 ng or less. or less, or about 5 ng or less, or about 1 ng or less of a cell-free nucleic acid molecule. The amount may be at least 1 fg, at least 10 fg, at least 100 fg, at least 1 pg, at least 10 pg, at least 100 pg, at least 1 ng, at least 10 ng, at least 100 ng, at least 150 ng, or at least 200 ng of a cell-free nucleic acid molecule. The amount may be 1 femtogram (fg), 10 fg, 100 fg, 1 picogram (pg), 10 pg, 100 pg, 1 ng, 10 ng, 100 ng, 150 ng, or 200 ng or less of a cell-free nucleic acid molecule. The method may comprise obtaining from 1 femtogram (fg) to 200 ng.

A cell-free nucleic acid is a nucleic acid that is not contained within a cell or is not otherwise associated with a cell, or that is, a nucleic acid that remains in a sample after removal of an intact cell. Cell-free nucleic acids include DNA, RNA, and hybrids thereof, including genomic DNA, mitochondrial DNA, siRNA, miRNA, circulating RNA (cRNA), tRNA, rRNA, small nucleolar RNA (snoRNA), Piwi-interacting RNA (piRNA), long non-coding RNA (long ncRNA), or fragments of any of these. Cell-free nucleic acids can be double-stranded, single-stranded, or hybrids thereof. Cell-free nucleic acids can be released into body fluids through secretion or cell death processes such as cellular necrosis and apoptosis. Some cell-free nucleic acids are released into body fluids from cancer cells, such as circulating tumor DNA (ctDNA). Others are released from healthy cells. In some embodiments, the cell-free nucleic acid is produced by a tumor cell. In some embodiments, the cell-free nucleic acid is produced by a mixture of tumor cells and non-tumor cells.

Cell-free nucleic acids exhibit a length distribution of, for example, about 100 to 500 nucleotides, and molecules of 110 to about 230 nucleotides make up about 90% of these molecules, a second minor peak in the range of 240 to 440 nucleotides is followed.

The cell-free nucleic acid can be isolated from a body fluid through a fractionation or split step, and cell-free nucleic acids as found herein in solution are separated from intact cells and other non-soluble components of body fluids. Spliting may include techniques such as centrifugation or filtration. Alternatively, cells in a body fluid can be lysed, and cell-free and cellular nucleic acids can be processed together. In general, after addition of buffer and washing steps, the nucleic acid can be precipitated with alcohol. Additional purification steps may remove contaminants or salts, such as using silica-based columns. Non-specific bulk carrier nucleic acids such as Cot-1 DNA, DNA or protein for bisulfite sequencing, hybridization, and/or ligation are added throughout the reaction, so that certain aspects of this procedure, such as yield, may be optimized.

After such processing, the sample may contain nucleic acids in various forms, including double-stranded DNA, single-stranded DNA, and single-stranded RNA. In some embodiments, single-stranded DNA and RNA can be converted to a double-stranded form, so that they are included in subsequent processing and analysis steps.

In one embodiment of the present invention, cfDNA may be derived from human genomic DNA, or it may be derived from DNA of cells, bacteria, fungi or viruses other than humans that coexist with humans or are infected with humans.

In one embodiment of the present invention, a method for screening a cancer-specific demethylation marker in cfDNA may comprise the following steps:
(a) treating cfDNA (cell free DNA) isolated from blood of a subject with methylation sensitive restriction enzymes;
(b) analyzing the sequence of each fragment;
(c) obtaining sequence information of a predetermined length from the N-terminus of the fragment;
(d) counting the frequency of each of the sequence information;
(e) screening cancer-specific sequence information as a cancer-specific demethylation marker in cfDNA.

Step (a) is a step of treating methylation sensitive restriction enzyme in cfDNA (cell free DNA) isolated from blood.

cfDNA is isolated from a subject. Preferably, cfDNA can be isolated from plasma. The isolation method may be performed by a conventional DNA isolation method known in the art, in which purity suitable for restriction enzyme treatment and sequencing can be obtained.

In one embodiment of the present invention, the methylation sensitive restriction enzyme is AatII, AclI, AgeI, Aor13HI I, AscI, AsiSI, AvaI, BsaHI, BsiEI, BsiWI, BspDI, BsrFI, BssHII, BstBI, ClaI, Cpo I, EagI, FseI, HaeII, HhaI, HinP1I, HpaII (or HapII), HpyCH4IV, Hpy99I, KasI, MluI, NarI, NgoMIV, NotI, PaeR7I, PluTI, PvuI, RsrII, SacII, SalI, SgrAI or TspMI. Preferably, the methylation-sensitive restriction enzyme of the present invention i) selectively cleaves unmethylated target regions, ii) the cleaved end makes a cohesive end (not a blunt end), so the conjugation efficiency of an adapter with a complementary cohesive end can be increased, so it can have a characteristic that can make a high-quality library.

In one embodiment of the present invention, the methylation-sensitive restriction enzyme is preferably an enzyme capable of selectively cleaving CpG methylation, that is, an enzyme capable of specifically cleaving a restriction enzyme recognition site including demethylated CpG. However, depending on how many restriction enzyme recognition sites exist in the genome, the analysis cost that requires coverage and sequencing of the entire genome may differ, so that an appropriate restriction enzyme can be selected according to the purpose.

**Table 1.**

| Enzyme | Recognition Site | SEQ ID NO | Enzyme | Recognition Site | SEQ ID NO |
|---|---|---|---|---|---|
| AatII | GACGT↓C | 1 | Hhal | GCG↓C | 20 |
| AclI | AA↓CGTT | 2 | HinP1I | G↓CGC | 21 |
| AgeI | A↓CCGGT | 3 | Hpall | C↓CGG | 22 |
| Aor13H I | T↓CCGGA | 4 | HpyCH4IV | A↓CGT | 23 |
| AscI | GG↓CGCGCC | 5 | Hpy99I | CGWCG↓ | 24 |
| AsiSI | GCGAT↓CGC | 6 | KasI | G↓GCGCC | 25 |
| AvaI | C↓YCGRG | 7 | MluI | A↓CGCGT | 26 |
| BsaHI | GR↓CGYC | 8 | NarI | GG↓CGCC | 27 |
| BsiEI | CGRY↓CG | 9 | NgoMIV | G↓CCGGC | 28 |
| BsiWI | C↓GTACG | 10 | NotI | GC↓GGCCGC | 29 |
| BspDI | AT↓CGAT | 11 | PaeR7I | C↓TCGAG | 30 |
| BsrFI | R↓CCGGY | 12 | PluTI | GGCGC↓C | 31 |
| BssHII | G↓CGCGC | 13 | PvuI | CGAT↓CG | 32 |
| BstBI | TT↓CGAA | 14 | RsrII | CG↓GWCCG | 33 |
| Clal | AT↓CGAT | 15 | SacII | CCGC↓GG | 34 |
| Cpo I | CG↓GWCCG | 16 | SalI | G↓TCGAC | 35 |
| Eagl | C↓GGCCG | 17 | SgrAI | CR↓CCGGYG | 36 |
| Fsel | GGCCGG↓CC | 18 | TspMI | C↓CCGGG | 37 |
| Haell | RGCGC↓Y | 19 | | | |

| | | | | | |
|---|---|---|---|---|---|
| (↓ indicates the restriction site) | | | | | |

Step (b) is a step of sequencing each fragment.

In one embodiment of the present invention, the sequence translation is performed by a sequence translation method known in the art. Sequence translation translates the sequence of each fragment cleaved or uncleaved by a methylation-sensitive restriction enzyme. Since the sequence translation reads a large number of fragments, preferably at least 10000 or more, at least 20000 or more, at least 30000 or more, at least 40000 or more, at least 50000 or more, at least 100000 or more, at least 1000000 or more fragments, so suitable sequencing methods for this are preferred.

For sequencing, a sequencing method known in the art may be used, but any capable sequencing method of a large amount of sequences in order to sequencing each fragment in sufficient quantity may be used without limitation. For example, if the next generation sequencing method (NGS) is used, it has the advantage that a large amount of sequences can be sequenced within 18 hours at a low cost, and the accuracy is very high when a sufficient amount of sequence is read, and the sequenced data can be analyzed qualitatively and quantitatively.

For sequence translation, preferably, an appropriate adapter may be attached so that only the DNA fragment cleaved by a methylation-sensitive restriction enzyme can be translated. DNA in the sample may or may not be cleaved by a methylation-sensitive restriction enzyme depending on the methylation state. For example, it is methylated in normal human cfDNA, but in the case of detecting cancered and demethylated cancer DNA, if only the demethylated and cleaved fragment can be sequenced, it becomes easy to detect cfDNA mixed in a very low ratio. Therefore, if an adapter having a structure complementary to the cohesive end produced by cleavage by a methylation-sensitive restriction enzyme is used, since a library is made only with cleaved fragments, it is possible to selectively interpret cancer-induced fragments in the translation stage.

Step (c) is a step of obtaining sequence information of a predetermined length from the 5'-end of the fragment.

In one embodiment of the present invention, the term 'predetermined length' indicates the length of a base or base pair from the 5'-end in each sequence-translated fragment, and may preferably be 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150 bases. Meanwhile, the predetermined length may be one of a natural number less than 25 and a natural number greater than 150 depending on a cancer to be screened or analyzed, a type of a sample, and the like. More preferably the predetermined length may be 30, 60, 80 or 90 bases. Also, in one embodiment of the present invention, the 'predetermined length' may be any natural number from 5 to 1000.

Step (d) is a step of counting the frequency of each of the sequence information.

In the obtained sequence information, the frequency of each sequence information starting at the 5' end is counted as the cohesive end sequence (CGG in the case of Hpall) generated by restriction enzyme cleavage. That is, counting the types (In the case of 30nt, 4³⁰ kinds of sequences are theoretically possible) of sequences of one predetermined length (for example 30) from all sequences obtained by sequencing one sample, counting the number of occurrences of each kind of sequence. The value of each sequence counted is normalized for comparison with the values of other samples. This normalization is to divide each aggregated value by a value proportional to the sequenced amount for a direct quantitative comparison between samples if the amount of readout for each sample is different. In this case, various values are possible, such as the total number of sequences translated in each sample and the number of sequences mapped to the house keeping gene region, as a value proportional to the amount of translation.

Step (e) is a step of screening cancer-specific sequence information as a cancer-specific demethylation marker in cfDNA.

In the normal sample group and the cancer sample group, counted and normalized values for each sequence combination of a predetermined length are compared to select a sequence of a predetermined length significantly higher in the cancer sample group as a marker. Most simply, in each predetermined length sequence combination, the difference between the average value of the normal sample group and the cancer sample group is used, or sequences with significant differences in the two sample groups are selected using various statistical techniques such as T-test, Mann-Whitney test, Wilcoxon Test, or Cohen's D test and the like. In this embodiment, for breast cancer and lung cancer, we analyzed the difference in mean values.

The screened cancer-specific demethylation marker may be a marker customized to the subject providing the sample, and may be a marker commonly applied to cancer type, stage, race, or family.

In one embodiment of the present invention, the cancer-specific demethylation marker in cfDNA is the sequence of the remaining region after the N-terminal sequence is cut among the recognition sites of the restriction enzyme, and it consists of a nucleotide sequence having the same length as the predetermined length. For example, the Hpall restriction enzyme recognizes the CCGG base and cuts between C and C. Thus, the N-terminus of the restricted fragment begins with CGG. Since the cancer-specific demethylation marker has a nucleotide sequence of a predetermined length, if the predetermined length is 30, it is selected from the sequences of CGGNNNNNNNNNNNNNNNNNNNNNNNNNNN (SEQ ID NO: 38) (N is any base, 30 bp). In this case, since N is 27, 4²⁷ (=18,014,398,509,481,984) fragments can theoretically exist, and cancer-specific demethylation markers are screened among them. If the predetermined length is 60, the length of the cancer-specific demethylation marker is 60 bases, and the predetermined length and the length of the cancer-specific demethylation marker are the same.

In addition, the present invention relates to a cancer diagnosis method comprising:
isolating cfDNA from blood isolated from a subject; treating the isolated cfDNA (cell free DNA) with methylation-sensitive restriction enzymes; analyzing the sequence of each fragment; obtaining sequence information of a predetermined length from the N-terminus of the fragment; counting the frequency of each of the sequence information; calculating the frequency of cancer-specific demethylation markers in cfDNA and determining cancer.

In one embodiment of the present invention, the subject is a patient in need of a diagnosis of cancer. In one embodiment of the present invention, the predetermined length is the same length as the cancer-specific demethylation marker in cfDNA. In one embodiment of the present invention, the cancer-specific demethylation marker in cfDNA is a marker set consisting of 1 to 50, preferably 3 to 40, more preferably 5 to 30 markers.

In addition, the present invention relates to a method of analyzing sequence information of a predetermined length at the N-terminus of a methylation-sensitive restriction enzyme fragment of cfDNA isolated from a subject to provide information necessary for cancer diagnosis.

In addition, according to the present invention, the N-terminus is a sequence of the cohesive end of the recognition site of a methylation-sensitive restriction enzyme (e.g., the sequence of CGG), consists of a sequence of 25 to 150 bases consecutively (preferably 30 bases, 35 bases, 40 bases, 45 bases or 50 bases), it provides a cancer-specific demethylation marker in cfDNA screened by the method of the present invention, the sequence of the cohesive end in this paragraph may be selected from the group consisting of ACGTC (SEQ ID NO: 39) , ATCG (SEQ ID NO: 40), ATCGC (SEQ ID NO: 41), CCGGA (SEQ ID NO: 42), CCGGC (SEQ ID NO: 43), CCGGCC (SEQ ID NO: 44), CCGGG (SEQ ID NO: 45), CCGGT (SEQ ID NO: 46), CCGGY (SEQ ID NO: 47), CCGGYG (SEQ ID NO: 48), CG (SEQ ID NO: 49), CGAA (SEQ ID NO: 50), CGAT (SEQ ID NO: 51), CGC (SEQ ID NO: 52), CGCC (SEQ ID NO: 53), CGCGC (SEQ ID NO: 54), CGCGCC (SEQ ID NO: 55), CGCGT (SEQ ID NO: 56), CGG (SEQ ID NO: 57), CGT (SEQ ID NO: 58), CGTT (SEQ ID NO: 59), CGWCG (SEQ ID NO: 60), CGYC ( SEQ ID NO: 61), GCGCC (SEQ ID NO: 62), GCGCY (SEQ ID NO: 63), GCGG (SEQ ID NO: 64), GGCCG (SEQ ID NO: 65), GGCCGC (SEQ ID NO: 66), GTACG (SEQ ID NO: 67), GWCCG (SEQ ID NO: 68), RYCG (SEQ ID NO: 69), TCGAC (SEQ ID NO: 70), TCGAG (SEQ ID NO: 71) and YCGRG (SEQ ID NO: 72). In this case, the indication of the base follows the standard notation, for example, A represents adenine, C is cytosine, T is thymine, G is guanine, Y is C or T, W is A or T, R is A or G.

In one embodiment of the present invention, cancer may be, but is not limited to, cervical cancer, lung cancer, pancreatic cancer, liver cancer, colon cancer, bone cancer, skin cancer, head or neck cancer, skin or intraocular melanoma, uterine cancer, ovarian cancer, rectal cancer, stomach cancer, anal cancer, colon cancer, breast cancer, fallopian tube carcinoma, endometrial carcinoma, vaginal carcinoma, vulvar carcinoma, esophageal cancer, small intestine cancer, endocrine adenocarcinoma, thyroid cancer, parathyroid cancer, adrenal cancer, soft tissue sarcoma, urethral cancer, penile cancer, prostate cancer, bladder cancer, kidney cancer or ureter cancer.

### Analysis for Diagnosis of Cancer Diseases

The diagnostic method can be used to diagnose the presence of a condition, particularly a disease, in a subject, or characterize the condition (e.g., to determine the staging of cancer or to determine the heterogeneity of cancer), or monitor the response to treatment of the condition, or prognosticate the risk of developing a condition or subsequent course of a condition. The present disclosure may also be useful in determining the efficacy of a particular therapy. In another example, a particular therapy may correlate with the genetic profile of the cancer over time. Such correlations may be useful in choosing a therapy. Additionally, if cancer is observed to be in remission after treatment, the diagnostic method can be used to monitor residual disease or recurrence of disease.

Genetic data can also be used to characterize specific forms of cancer. Cancers are often heterogeneous in both composition and stage. Genetic profile data may allow for the characterization of specific subtypes of cancer, which may be important in diagnosing or treating cancers of that specific subtype. Such information may also provide subject or practitioner clues regarding the prognosis of a specific type of cancer, and allow the subject or practitioner to adopt treatment options as the disease progresses. Some cancers can progress to become more aggressive and genetically unstable. Other cancers may remain benign, inactive or dormant. The systems and methods of the present disclosure may be useful in determining disease progression.

### Markers and Panels

The present invention uses each marker individually as a diagnostic or predictive marker, or can be used as a panel display form by combining several markers, and several markers can be identified to improve reliability and efficiency through the overall pattern or list of methylated sites. The markers identified in the present invention may be used individually or as a combined marker set. Markers can be ranked, weighted, and level of likelihood of developing disease according to the number and importance of markers methylated together. Such an algorithm belongs to the present invention.

### Substrate

The target nucleic acid site can be hybridized with a known probe immobilized on a solid support (substrate).

As used herein, "substrate" is a mean of a mixture comprising a substance, structure, surface or material, a material of abiotic, synthetic, inanimate, planar, spherical or specific binding, a planar surface, it may include a number of other recognition sites beyond hybridization or enzyme recognition sites or many other recognition sites or numerous other molecular species composed of a surface, structure or material. The substrate may be, for example, a semiconductor, an (organic) synthetic metal, a synthetic semiconductor, an insulator and a dopant; metals, alloys, elements, compounds and minerals; synthesized, disassembled, etched, lithographed, printed, microfabricated slides, devices, structures and surfaces; industrial polymers, plastics, membranes, silicones, silicates, glass, metals and ceramics; wood, paper, cardboard, cotton, wool, cloth, woven and non-woven fibers, materials and fabrics, but is not limited thereto.

Some types of membranes are known in the art to have adhesion to nucleic acid sequences. A specific, non-limiting example of such a membrane is a membrane for detecting gene expression, such as a commercially used membrane such as nitrocellulose or polyvinyl chloride, diazotized paper and trade name GENESCREEN, trade name ZETAPROBE and trade name NYTRAN and the like. Also beads, glass, wafers and metal substrates are included. Methods for adhesion nucleic acids to such objects are well known in the art. Alternatively, screening can also be carried out in the liquid phase.

### ADVANTAGEOUS EFFECT

Therefore, the method of the present invention can screen cancer-specific demethylation markers in cfDNA, and the screened marker can provide information necessary for diagnosis of cancer, monitoring of a treatment regimen, and prognosis of a cancer patient, and thus can be usefully used for anti-cancer treatment.

### BRIEF DESCRIPTION OF THE DRAWINGS

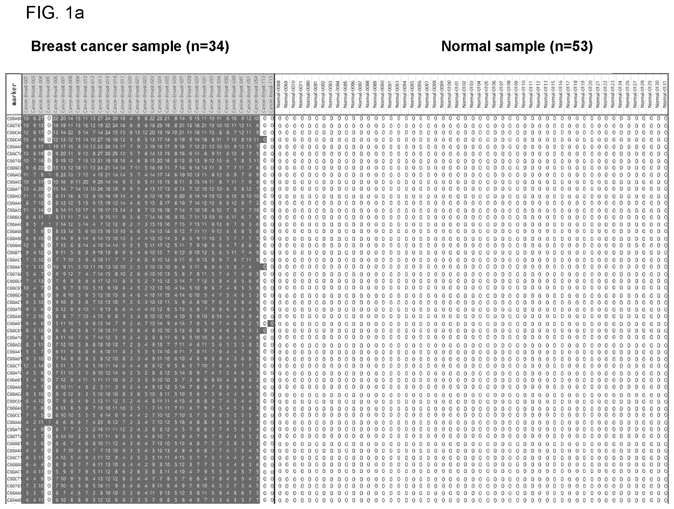

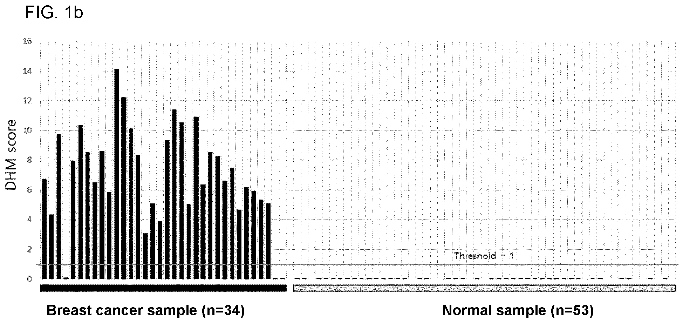

FIG. 1a is an example of the results of analysis by treatment with HpaII in a breast cancer patient sample group and a normal sample group, and FIG. 1b is a schematic view thereof.

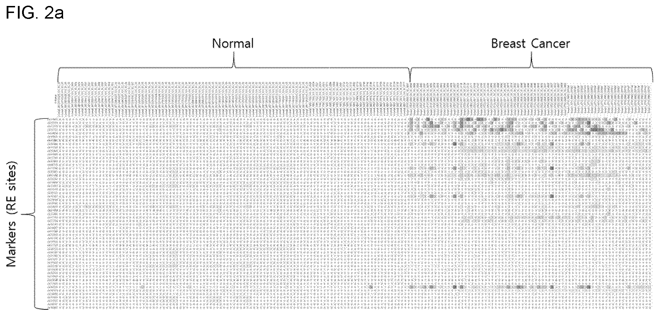

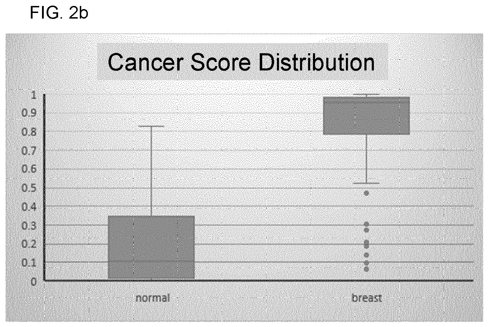

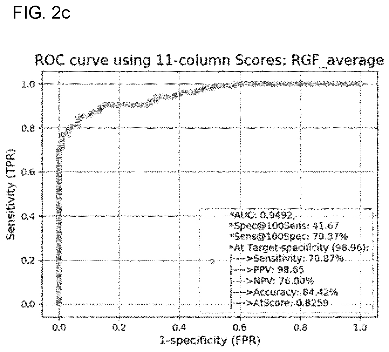

FIG. 2a shows regions with statistically significant differences between the two sample groups by standardizing (z-score) the number of reads mapped to enzyme restriction sites treated with Sacll in a breast cancer patient sample group and a normal sample group. FIG. 2b is a diagram illustrating a difference in probability values between a normal group and a breast cancer group by creating a machine learning model using the breast cancer-specific markers extracted in the process of FIG. 2a to calculate breast cancer prediction probability values (0.0 to 1.0). FIG. 2c shows the ROC (Receiver Operator Characteristic) curve through the average probability value of each test sample by repeating the model learning 20 times, and AUC (Area Under Curve: 0.0 to 1.0) values are expressed.

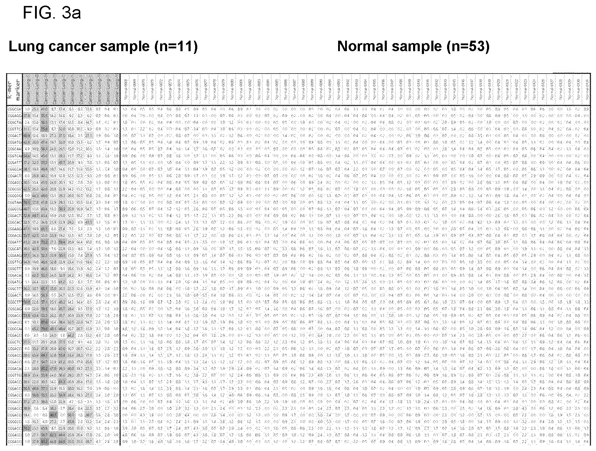

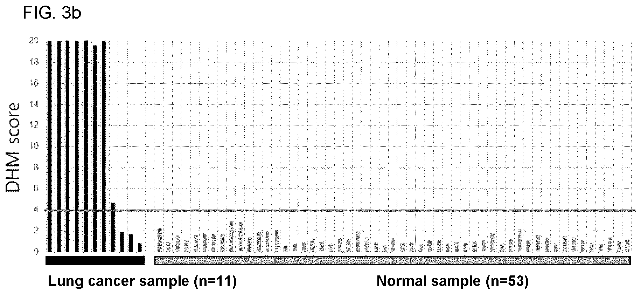

FIG. 3a is an example of the results of analysis by treatment with Hpall in the breast cancer patient sample group and the normal sample group, FIG. 3b is a schematic view thereof.

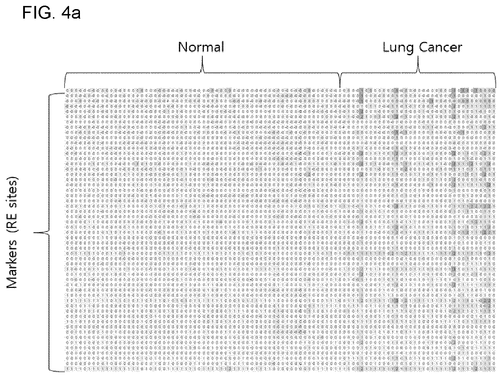

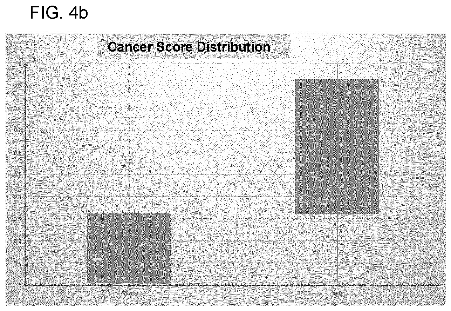

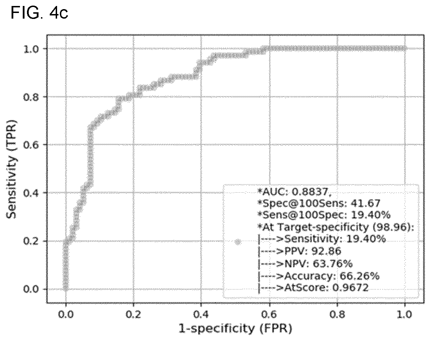

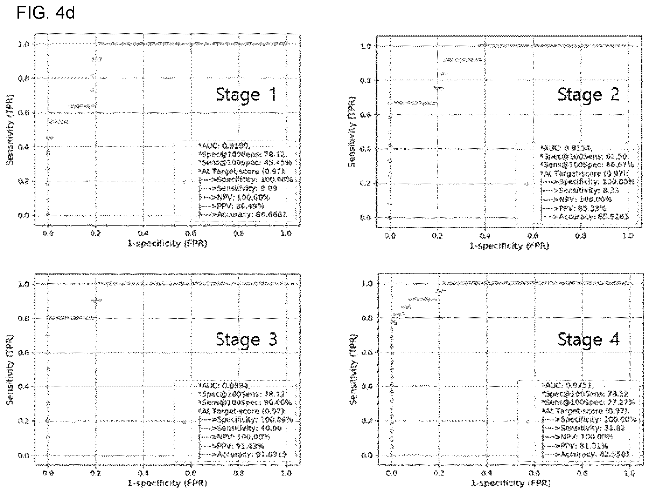

FIG. 4a shows regions with statistically significant differences between the two sample groups by standardizing (z-score) the number of reads mapped to the enzyme restriction site treated with Sacll in the lung cancer patient sample group and the normal sample group. FIG. 4b is a diagram illustrating the difference in probability values between the normal group and the lung cancer group by creating a machine learning model using the lung cancer-specific markers extracted in the process of FIG. 4a to calculate the lung cancer prediction probability values (0.0 to 1.0). FIG. 4c shows the ROC (Receiver Operator Characteristic) curve through the average probability value of each test sample by repeating the model learning 20 times, and AUC (Area Under Curve: 0.0 to 1.0) values are expressed. FIG. 4d is a diagram of the ROC curve for each stage of lung cancer classification.

### MODE FOR CARRYING OUT INVENTION

Hereinafter, the present invention will be described in detail.

However, the following examples only illustrate the present invention, and the content of the present invention is not limited to the following examples.

### Experiment method

### 1. Isolation of cfDNA from blood

Breast cancer patients (n=102), lung cancer patients (n=75) and healthy people (n=139) were subjected to blood collection in cfDNA-only blood collection tubes. The separated blood was centrifuged at 2000 rpm for 20 minutes to separate plasma. The separated plasma (supernatant) was transferred to a 1.5 ml tube and centrifuged at 16000 rpm for 10 minutes. Thereafter, cfDNA was isolated using Chemagen cfDNA prep equipment according to the manufacturer's instructions.

### 2. Library production

After making the end of the isolated cfDNA into a blunt end, A tailing was induced, and ligation of the p7 adapter and cfDNA was induced here. Restriction enzyme Hpall was treated to cut the demethylated CCGG site. At this time, in the case of methylated CpG, it is not restricted by Hpall. A p5 adapter having an Hpall sticky end was added thereto, and the cfDNA fragment cleaved by Hpall was ligated, and this was used as an analysis library.

### 3. Analysis of sequence information

The analysis library for each sample was used as NGS to obtain sequence information for each sequence included in the library. From the decoded sequence of each sample, a sequence starting with a restriction enzyme recognition sequence (CGG for Hpall, GC for Sacll) was selected, and sequence information of a certain length (ex. 30, 60, 80, etc.) was obtained from 5' of the selected sequences and the sequences were classified according to the predetermined length. The frequencies of classified sequences in each sample were counted and normalized for comparison between samples. For each sequence candidate, significantly higher read sequences were identified in the cancer sample group compared to the normal sample group, at this time, since the sequence was specifically demethylated in the cancer sample group, it could be selected as a demethylation marker (In the case of Hpall; methylation markers in the case of restriction enzymes that cut methylation sites). An average value (DHM score) is obtained for each sample for a given marker, and a reference value of the DHM score that can distinguish a cancer sample from a normal sample is determined. This DHM score was used for the judgment of the sample.

### 4. Examination of samples and determination of cancer occurrence

The unknown sample was sequenced and analyzed according to the above-described experimental method and analysis method. Obtaining the DHM score, which is the average of the values corresponding to the markers selected in the analysis of the sequence information (Item 3), if it was higher than the predetermined DHM reference value, it was determined that there was cancer.

### Example 1: Screening of cancer-specific demethylation markers for breast cancer cfDNA and determination accuracy test using the same

cfDNA isolated from 34 breast cancer sample groups and 53 normal samples was restricted with Hpall, one of the methylation-sensitive restriction enzymes, and sequencing and analysis were performed according to the above-described experimental method. Among the translated sequences, the sequence of the first 80 nt of the sequence starting with CGG was counted and compared as marker candidates. 173 markers with an average value of 5 or more for each marker in breast cancer and 10 times or more than the average value of the normal group were selected as markers, and the DHM score, which is the average of these 173 values in each sample, was obtained.

As a result, shown in FIG. 1, a table was created in which normalized scores for each marker of breast cancer and normal samples were recorded, to make it easier to see the score, it is expressed as a heatmap with high numbers in red and low numbers in blue. FIG. 1a shows the upper part of the 173 markers, among the selected markers, it could be seen that 31 samples except for three samples had a value above a certain level in the breast cancer sample, whereas all samples had a value close to 0 in the normal sample.

FIG. 1b is a bar graph showing the DHM score, which is an average of 173 marker values of each sample shown in FIG. 1a. Based on the DHM score 1, it can be seen that breast cancer and normal samples are clearly distinguished.

As a result of comparing the DHM score of the breast cancer group and the normal group, when the DHM score is set to 1, 31 out of 34 breast cancer samples are judged as breast cancer, all 53 out of 53 normal samples could be judged as normal. As a result of calculating the accuracy, the sensitivity was 91.2% and the specificity was 100% (see FIGS. 1a and 1b).

### Example 2: Screening of cancer-specific demethylation markers for breast cancer cfDNA treated with SacII and determination accuracy test using the same

cfDNA isolated from 102 breast cancer samples and 139 normal samples was restricted with Sacll, a methylation-sensitive restriction enzyme, and sequencing and analysis were performed according to the above-described experimental method. Among the decoded sequences, the sequence of the first 80 nt of the sequence starting with GC was counted and compared as marker candidates.

Each marker is normalized through the IQR (InterQuartile Range) average value and standardized through the Z-score to reduce the difference that may appear between sequencing. Afterwards, for each marker, a t-test is performed between the breast cancer group and the normal group to select a marker whose p-value is below a specific threshold (e.g., 10⁻⁵), and the final DHM Score is calculated through the selected marker. The final score can be calculated by simply adding the corresponding values for each sample for the selected marker, it can be calculated as a predictive probability value by creating a classification model of machine learning such as logistic regression analysis.

As a result, shown in FIG. 2a, a table was created in which normalized / standardized values for each marker of breast cancer and normal samples were recorded, to make it easier to see the score, it is expressed as a heatmap with high numbers in red and low numbers in green. FIG. 2b was confirmed that there was a clear difference in the distribution of probability values between the breast cancer group and the normal group by creating a machine learning prediction model through the selected marker and by creating a result value with probability values from 0 to 1.

In FIG. 2c, using K-Fold Cross Validation among the machine learning model testing methods, random extraction of training and test groups is performed at 8:2 for each cycle, the result value is calculated through 20 different training data for one sample by repeating this operation 20 times, the average value was taken and the ROC (Receiver Operating Characteristic) curve was drawn to measure the performance.

As a result of comparing the DHM scores of the breast cancer group and the normal group, it was found that the AUC was 0.9492, and the sensitivity was 70.87% based on the specificity 100% (see FIG. 2c).

### Example 3: Screening of cancer-specific demethylation markers for lung cancer cfDNA and determination accuracy test using the same

When the lung cancer group of 11 samples and the 53 normal sample group were compared with a length of 30 nt, there were 198 markers with values 5 times or more and 157 markers with values 10 times or more in the lung cancer group than the average value of the normal group. The DHM score was obtained by adding up all of these 198 values in each sample.

As a result, shown in FIG. 3a, a table was created that recorded the normalized scores for each marker of lung cancer and normal samples, to make it easier to see the score, it is expressed as a heatmap with high numbers in red and low numbers in blue. FIG. 3a shows the top part of the 198 markers. In the selected marker, 8 samples except for 3 samples had values above the reference value in the lung cancer sample, whereas all samples had a value of 3 or less, which was lower than the reference value 4 in the normal sample.

FIG. 3b is a bar graph showing the DHM score, which is the average of 198 marker values of each sample shown in FIG. 3a. Based on the DHM score of 4, lung cancer and normal samples were clearly distinguished.

As a result of comparing the DHM scores of the lung cancer group and the normal group, if the DHM score is based on 4, 8 out of 11 lung cancer samples are judged as lung cancer, and all 53 out of 53 normal samples could be judged as normal. As a result of calculating the accuracy, the sensitivity was 72.7% and the specificity was 100% (see FIGS. 3a and 3b).

### Example 4: Screening of cancer-specific demethylation markers for lung cancer cfDNA treated with SacII and determination accuracy test using the same

cfDNA isolated from 75 lung cancer samples and 129 normal samples was restricted with Sacll, one of a methylation-sensitive restriction enzyme, sequencing and analysis were performed according to the above-described experimental method. Among the decoded sequences, the sequence of the first 80 nt of the sequence starting with GC was counted and compared as marker candidates.

Each marker is normalized through the IQR (InterQuartile Range) average value and standardized through the Z-score to reduce the difference that may appear between sequencing. Thereafter, for each marker, a t-test was performed between the lung cancer group and the normal group, and select a marker whose p-value is below a certain threshold (e.g., 10⁻⁵), the final DHM Score is calculated through the selected marker. The final score can be calculated by simply adding the corresponding values for each sample for the selected marker, it can be calculated as a predictive probability value by creating a classification model of machine learning such as logistic regression analysis.

As a result, shown in FIG. 4a, a table was created in which normalized / standardized values for each marker of lung cancer and normal samples were recorded, to make it easier to see the score, it is expressed as a heatmap with high numbers in red and low numbers in green. FIG. 4b was confirmed that there was a clear difference in the distribution of probability values between the breast cancer group and the normal group by creating a machine learning prediction model through the selected marker and by creating a result value with probability values from 0 to 1.

In FIG. 4c, using K-Fold Cross Validation among the machine learning model testing methods, random extraction of training and test groups is performed at 8:2 for each cycle, the result value is calculated through 20 different training data for one sample by repeating this operation 20 times, the average value was taken and the ROC (Receiver Operating Characteristic) curve was drawn to measure the performance. FIG. 4d shows the accuracy of samples classified according to each stage of lung cancer.

As a result of comparing the DHM score of the lung cancer group and the normal group, it was found that the AUC was 0.8837, the sensitivity was 41.67% based on the specificity 100% (see FIG. 4c).

### INDUSTRIAL APPLICABILITY

As described above, the method of the present invention can screen cancer-specific demethylation markers in cfDNA, and the screened marker can provide information necessary for diagnosis of cancer, monitoring of a treatment regimen, and prognosis of a cancer patient, and thus can be usefully used for anticancer treatment.

## Claims

1. A method of screening a cancer-specific demethylation marker in cfDNA comprising:
(a) treating cfDNA (cell free DNA) isolated from a subject with methylation sensitive restriction enzymes;
(b) analyzing the sequence of each fragment;
(c) obtaining sequence information of a predetermined length from the N-terminus of the fragment;
(d) counting the frequency of each of the sequence information;
(e) screening cancer-specific sequence information as a cancer-specific demethylation marker in cfDNA.

2. The method according to claim 1, wherein the methylation-sensitive restriction enzyme is selected from the group consisting of AatII, AclI, AgeI, Aor13H I, AscI, AsiSI, AvaI, BsaHI, BsiEI, BsiWI, BspDI, BsrFI, BssHII, BstBI, ClaI, Cpo I, EagI, FseI, HaeII, HhaI, HinP1I, HpaII, HpyCH4IV, Hpy99I, KasI, MluI, NarI, NgoMIV, NotI, PaeR7I, PluTI, PvuI, RsrII, SacII, SalI, SgrAI and TspMI.

3. The method according to claim 1, wherein analyzing the sequence is performed by next-generation sequencing (NGS).

4. The method according to claim 1, wherein the predetermined length is a base of any one length selected from the group consisting of 25, 26, 27, 28, 29, 30, 31, 32, 33, 34, 35, 36, 37, 38, 39, 40, 41, 42, 43, 44, 45, 46, 47, 48, 49, 50 , 51, 52, 53, 54, 55, 56, 57, 58, 59, 60, 61, 62, 63, 64, 65, 66, 67, 68, 69, 70, 71, 72, 73, 74, 75 , 76, 77, 78, 79, 80, 81, 82, 83, 84, 85, 86, 87, 88, 89, 90, 91, 92, 93, 94, 95, 96, 97, 98, 99, 100 , 101, 102, 103, 104, 105, 106, 107, 108, 109, 110, 111, 112, 113, 114, 115, 116, 117, 118, 119, 120, 121, 122, 123, 124, 125 , 126, 127, 128, 129, 130, 131, 132, 133, 134, 135, 136, 137, 138, 139, 140, 141, 142, 143, 144, 145, 146, 147, 148, 149 and 150.

5. The method according to claim 1, wherein the N-terminal sequence of the cancer-specific demethylation marker in the cfDNA is a cohesive end sequence of the recognition site of the restriction enzyme, and wherein the nucleotide sequence consists of the same length as the predetermined length.

6. The method according to claim 1, wherein the cancer is selected from the group consisting of cervical cancer, lung cancer, pancreatic cancer, liver cancer, colon cancer, bone cancer, skin cancer, head or neck cancer, skin or intraocular melanoma, uterine cancer, ovarian cancer, rectal cancer, stomach cancer, anal cancer, colon cancer, breast cancer, fallopian tube carcinoma, endometrial carcinoma, vaginal carcinoma, vulvar carcinoma, esophageal cancer, small intestine cancer, endocrine adenocarcinoma, thyroid cancer, parathyroid cancer, adrenal cancer, soft tissue sarcoma, urethral cancer, penile cancer, prostate cancer, bladder cancer, kidney cancer and ureter cancer.

7. A cancer diagnosis method comprising:
(a) treating cfDNA (cell free DNA) isolated from a subject with methylation-sensitive restriction enzymes;
(b) analyzing the sequence of each fragment;
(c) obtaining sequence information of a predetermined length from the N-terminus of the fragment;
(d) counting the frequency of each of the sequence information;
(e) calculating the frequency of cancer-specific demethylation markers in cfDNA and determining cancer.

8. The method according to claim 7, wherein the subject is a patient in need of cancer diagnosis.

9. The method according to claim 7, wherein the predetermined length is the same as a cancer-specific demethylation marker in the cfDNA.

10. The method according to claim 7, wherein the cancer-specific demethylation marker in the cfDNA is a marker set consisting of 5 to 50 markers.

11. A method of analyzing sequence information of a predetermined length at the N-terminus of a methylation-sensitive restriction enzyme fragment of cfDNA isolated from a subject to provide information necessary for cancer diagnosis.

12. A cancer-specific demethylation marker in cfDNA selected by the method of claim 1, wherein the N-terminus of the cancer-specific demethylation marker is the sequence of the cohesive end of the recognition site of a methylation-sensitive restriction enzyme, and consists of a sequence of 25 to 150 bases.

13. The cancer-specific demethylation marker according to claim 12, wherein the sticky end sequence is selected from the group consisting of ACGTC (SEQ ID NO: 39) , ATCG (SEQ ID NO: 40), ATCGC (SEQ ID NO: 41), CCGGA (SEQ ID NO: 42), CCGGC (SEQ ID NO: 43), CCGGCC (SEQ ID NO: 44), CCGGG (SEQ ID NO: 45), CCGGT (SEQ ID NO: 46), CCGGY (SEQ ID NO: 47), CCGGYG (SEQ ID NO: 48), CG (SEQ ID NO: 49), CGAA (SEQ ID NO: 50), CGAT (SEQ ID NO: 51), CGC (SEQ ID NO: 52), CGCC (SEQ ID NO: 53), CGCGC (SEQ ID NO: 54), CGCGCC (SEQ ID NO: 55), CGCGT (SEQ ID NO: 56), CGG (SEQ ID NO: 57), CGT (SEQ ID NO: 58), CGTT (SEQ ID NO: 59), CGWCG (SEQ ID NO: 60), CGYC ( SEQ ID NO: 61), GCGCC (SEQ ID NO: 62), GCGCY (SEQ ID NO: 63), GCGG (SEQ ID NO: 64), GGCCG (SEQ ID NO: 65), GGCCGC (SEQ ID NO: 66), GTACG (SEQ ID NO: 67), GWCCG (SEQ ID NO: 68), RYCG (SEQ ID NO: 69), TCGAC (SEQ ID NO: 70), TCGAG (SEQ ID NO: 71) and YCGRG (SEQ ID NO: 72).
